# EUROPÄISCHE PATENTSCHRIFT

(11) **EP 1 943 975 B1**
(45) Veröffentlichungstag und Bekanntmachung des Hinweises auf die Patenterteilung: **06.10.2010**
(21) Anmeldenummer: 07122079.2
(22) Anmeldetag: 03.12.2007
(51) Int. Cl.: A61B 19/02, A61B 19/00

(54) **Behälter zur Aufnahme von chirurgischen oder medizinischen Gegenständen mit Packschablone**
Holder for storage of surgical or medical equipment with filling template
Récipient destiné à la saisie d'articles chirurgicaux ou médicaux avec gabarit de remplissage

(30) Priorität: 15.01.2007 DE 102007003222
(43) Veröffentlichungstag der Anmeldung: 16.07.2008
(73) Patentinhaber: Aesculap AG, 78532 Tuttlingen (DE)
(72) Erfinder: Schuster, Stefan, 78048, Villingen-Schwenningen (DE); Oertmann, Friedrich-Wilhelm, 78532, Tuttlingen (DE)
(74) Vertreter: Hoeger, Stellrecht & Partner Patentanwälte

(56) Entgegenhaltungen:
- WO-A-2005/107525
- DE-A1- 19 749 869
- DE-U1-202007 000 934
- US-A- 4 828 113
- US-A- 5 379 887
- US-A- 6 158 437

## Beschreibung

Die Erfindung betrifft einen Behälter zur Aufnahme von chirurgischen oder medizinischen Gegenständen nach Anspruch 1.

Chirurgische und medizinische Gegenstände, beispielsweise chirurgische Instrumente, werden üblicherweise in Behältern aufbewahrt, die eine größere Anzahl derartiger Gegenstände aufnehmen können, beispielsweise einen kompletten Set von chirurgischen Instrumenten für eine bestimmte Operation. Die Gegenstände werden in dem Behälter in Halterungen in definierter Position gelagert, und die Behälter dienen nicht nur der Aufbewahrung der Gegenstände, sondern werden häufig auch bei der Reinigung und Sterilisation der Gegenstände eingesetzt. Dabei bleiben die Gegenstände in der Lagerungsposition im Behälter, und der Behälter wird von einem Reinigungsmedium oder Sterilisa-tionsmedium durchströmt. Dies kann über Öffnungen im Behälter erfolgen, beispielsweise über durchbrochene Bodenflächen und bei abgenommenem Deckel.

Um die Befüllung der Behälter mit den Gegenständen zu erleichtern und um kontrollieren zu können, ob die Befüllung vollständig und korrekt ist, ist es bekannt, Packschablonen zu verwenden, die entweder die einzulegenden Gegenstände naturgetreu in der erwünschten Position darstellen oder aber auch in stilisierter Form. Wenn diese Packschablonen auf den Boden des Behälters aufgelegt werden, können sich Schwierigkeiten beim Reinigen und Sterilisieren ergeben, da der Boden dann verschlossen ist und nicht für die Durchströmung zur Verfügung steht. Wenn andererseits größere Durchbrechungen im Boden angebracht werden, um die Durchströmung zu gewährleisten, müssen derartige Durchbrechungen auch in der Packschablone vorgesehen werden, und dann steht nur ein geringer Bereich der Packschablone für die Kennzeichnung der Instrumente und Gegenstände zur Verfügung.

US 5 379 887 zeigt die Merkmale des Oberbegriffs von Anspruch 1.

Es ist Aufgabe der Erfindung, einen gattungsgemäßen Behälter so auszubilden, dass einerseits der Benutzer in einfacher Weise die Packschablone sehen und bei der Befüllung des Behälters verwenden kann, während andererseits die Packschablone die Durchströmung des Behälters nicht behindert.

Diese Aufgabe wird bei einem Behälter der eingangs beschriebenen Art erfindungsgemäß durch die Merkmale des Anspruchs 1 gelöst. Dabei ist die Packschablone an der Innenseite des Deckels angeordnet. Auf diese Weise bleibt der Boden frei von der Packschablone, und der Behälter kann nach Abnahme des Deckels ungehindert von einem Reinigungs- oder Sterilisationsmedium durchströmt werden.

Die Packschablone an der Innenseite des Behälters ist für den Benutzer ohne weiteres zu erkennen, wenn er beispielsweise den Deckel vom Behälter abnimmt und mit der Innenseite nach oben neben den Behälter legt. Möglich wäre es auch, bei Behältern, bei denen der Deckel über ein Scharnier mit dem Unterteil verbunden ist, den Deckel aufzuklappen, so dass die Innenseite nach oben gerichtet ist und der Benutzer die Packschablone neben dem Unterteil sieht.

Günstig ist es, wenn sich die Packschablone über die gesamte Fläche des Deckels erstreckt, so dass die Gegenstände im Maßstab 1:1 in der gewünschten Position dargestellt werden können.

Bei einer besonders bevorzugten Ausführungsform ist vorgesehen, dass die Darstellung der Gegenstände auf der Packschablone unterschiedliche Farben aufweisen. Diese unterschiedlichen Farben können beispielsweise signalisieren, in welcher Reihenfolge die Gegenstände in den Behälter eingelegt oder auf diesem entnommen werden sollen, es ist auch möglich, dadurch bestimmte Gegenstände zu kennzeichnen, die zusammen gehören und beispielsweise bei der Operation gleichzeitig entnommen werden müssen.

Bei einem nicht erfindungsgemäßen Beispiel ist vorgesehen, dass der Boden des Behälters durchbrochen ist und dass das Unterteil in einer definierten Lage in den Deckel derart einsetzbar ist, dass die Packschablone auf der Innenseite des Deckels unterhalb des durchbrochenen Bodens mit der Packschablone nach oben weisend angeordnet ist.

Auf diese Weise kann der Benutzer durch den durchbrochenen Boden hindurch die Packschablone sehen und sich an der Darstellung der Gegenstände orientieren.

Bei allen erfindungsgemäßen Ausführungsformen ist vorgesehen , dass der Boden des Behälters von den Seitenwänden lösbar ist, dass die Gegenstände an Halterungen im Behälter gehalten sind, die an den Seitenwänden des Unterteils festgelegt sind, und dass das Unterteil nach Abtrennung des Bodens in einer definierten Lage in den Deckel derart einsetzbar ist, dass die Packschablone auf der Innenseite des Deckels unterhalb des Unterteils mit der Packschablone nach oben weisend angeordnet ist. In diesem Falle ist also kein durchbrochener Boden mehr vorgesehen, sondern die Baueinheit aus den Seitenwänden und den Halterungen für die Gegenstände wird unmittelbar in den umgekehrten Deckel eingesetzt, so dass die Packschablone an der offenen Unterseite sichtbar ist. Zum Reinigen und Sterilisieren wird der Deckel dann von der Baueinheit wieder abgenommen.

Es ist günstig, wenn sich das Unterteil beim Einsetzen in den Deckel mit diesem durch eine Rast- oder Klemmverbindung lösbar verbindet, so dass das in den Deckel eingesetzte Unterteil handhabbar ist, ohne dass sich der Deckel von alleine löst. Das Ablösen erfolgt entweder durch Lösen einer Rastverbindung oder durch Aufbringen einer bestimmten Kraft, die die Klemmkraft überwindet.

Die nachfolgende Beschreibung bevorzugter Ausführungsformen der Erfindung dient im Zusammenhang mit der Zeichnung der näheren Erläuterung. Es zeigen:

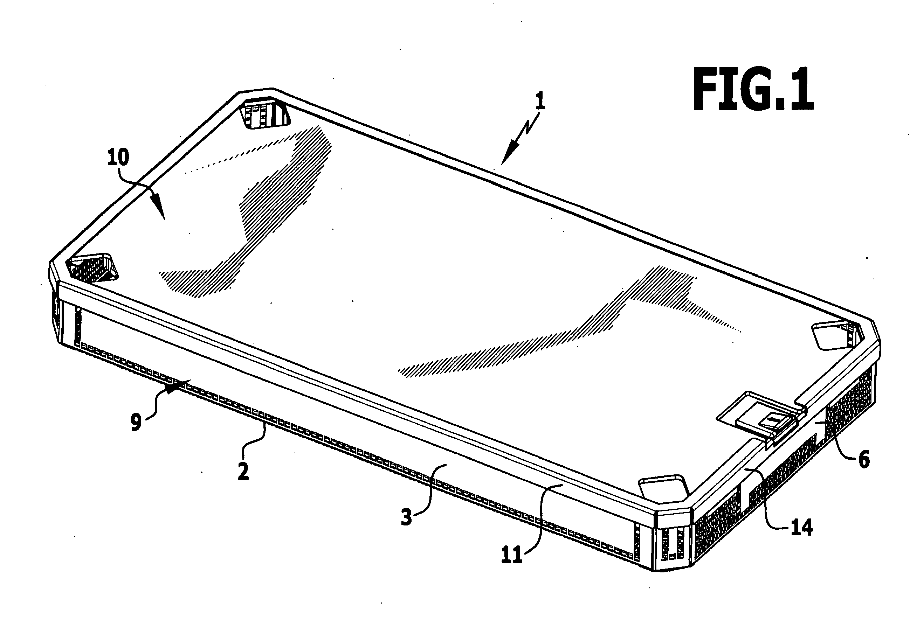

- Figur 1:: eine perspektivische Ansicht eines Behälters mit aufgesetztem und das Unterteil des Behälters verschließendem Deckel;

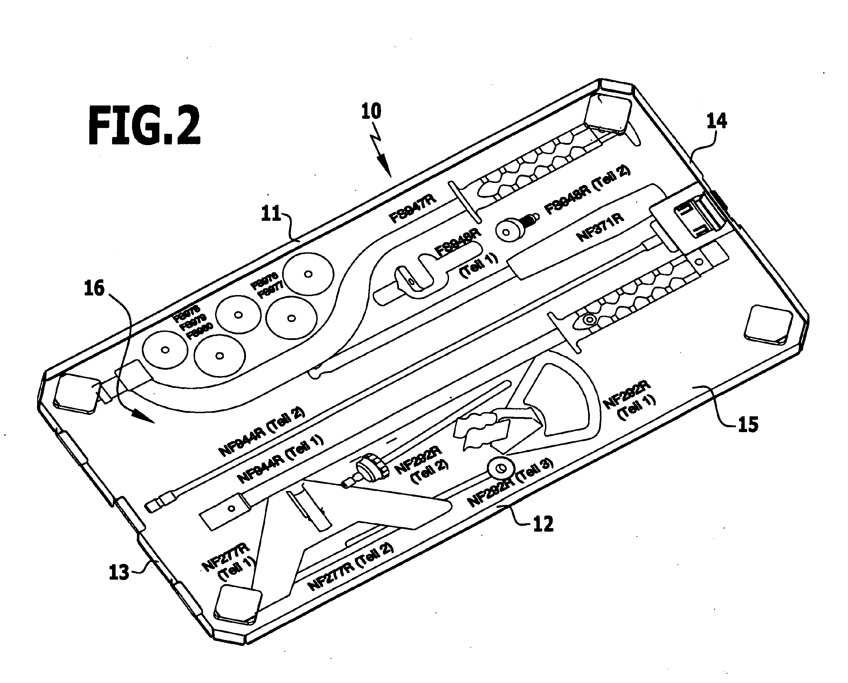

- Figur 2:: eine perspektivische Ansicht des abgenommenen Deckels von der Innenseite des Deckels her gesehen und
- Figur 3:: eine perspektivische Ansicht eines leeren Behälters mit durchbrochenem Boden, der in einen Deckel eingesetzt ist.

Der in der Zeichnung dargestellte Behälter 1 weist einen ebenen, im Wesentlichen rechteckigen Boden 2 auf, an den sich senkrechte Seitenwände 3, 4, 5, 6 anschließen. Der Boden 2 ist durchbrochen ausgeführt, beispielsweise nach Art eines Netzes oder Gitters mit einer Vielzahl von Öffnungen 7, die durch schmale Stege 8 voneinander getrennt sind.

An den Seitenwänden 3, 4, 5, 6 sind in der Zeichnung nicht dargestellte Halterungen angeordnet, auf die Instrumente oder andere medizinische Gegenstände aufgelegt werden können und die dadurch in definierter Lage in dem Behälter gelagert werden.

Der Boden 2, die Seitenwände 3, 4, 5, 6 und die Halterungen bilden gemeinsam ein Unterteil 9 des Behälters 1 aus. Dieses Unterteil 9 ist wannenförmig ausgebildet und an der Oberseite offen. Auf diese offene Oberseite kann ein Deckel 10 aufgesetzt werden, der rechteckförmig und eben ausgebildet ist und das gesamte Unterteil 9 überdeckt und der mit senkrecht abstehenden Rändern 11, 12, 13, 14 das Unterteil 9 umgreift, wenn er auf dieses aufgesetzt ist. Auf diese Weise verschließt der Deckel 10 das Unterteil 9 des Behälters, gegebenenfalls unter Zwischenlage einer flexiblen Dichtung.

Der Deckel 10 weist eine ebene, durchbrechungsfreie Oberseite auf, gegebenenfalls können Einprägungen 15 vorgesehen sein zur sicheren Lagerung von übereinander gestapelten Behältern gleicher Bauart.

Auf der Außenseite des Deckels 10 können Aufschriften angebracht werden, beispielsweise Firmenaufschriften oder Hinweise auf den Inhalt des Behälters, etwa durch Verwendung bestimmter Farbcodierungen etc.

Auf der Innenseite 15 des Deckels 10 ist eine sich über die gesamte Innenseite 15 erstreckende Packschablone 16 angeordnet, beispielsweise in Form eines Aufdruckes auf der Innenseite 15 oder in Form eines flächigen Bildträgers. Dabei kann es sich um eine Kunststofffolie, eine Papierfolie oder auch eine Kunststoffplatte handeln. Gemeinsam ist allen Packschablonen 16, dass auf ihnen die in dem Behälter zu lagernden Gegenstände dargestellt sind, und zwar in der Lage, in der sie in den Behälter 1 aufgenommen werden sollen.

Vorzugsweise ist der Maßstab dabei 1:1, es wäre aber grundsätzlich auch möglich, einen anderen Maßstab zu wählen und die Darstellung der Gegenstände nur auf einen Teilbereich der Innenseite 15 zu beschränken.

Die Darstellung kann eine naturgetreue Darstellung sein, etwa in Form einer Fotografie oder einer fotografieähnlichen Abbildung, insbesondere auch einer die Dreidimensionalität der Gegenstände berücksichtigenden Darstellung, es kann aber auch eine schematische Wiedergabe der zu lagernden Gegenstände auf der Packschablone dargestellt sein. Wesentlich ist lediglich, dass ein Benutzer anhand der Packschablone die Art der Gegenstände und ihre Anordnung in dem Behälter erkennen kann.

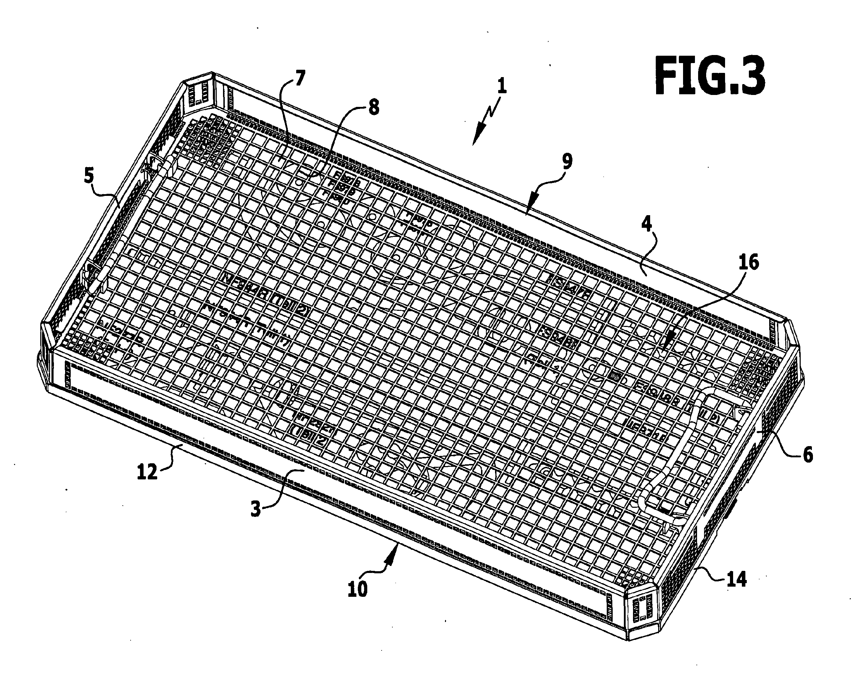

Zum Einlegen der Gegenstände oder zur Kontrolle der Vollständigkeit der Füllung des Behälters kann der Deckel mit der Innenseite 15 nach oben neben das Unterteil 9 gelegt werden, es kann aber auch in der aus Figur 3 ersichtlichen nicht erfindungsgemäßen Weise das Unterteil 9 in den mit der Packschablone 16 nach oben weisenden Deckel 10 eingesetzt werden, wobei die Rändern 11, 12, 13, 14 des Deckels 10 dann im unteren Bereich an den Seitenwänden 3, 4, 5 beziehungsweise 6 anliegen. Auf diese Weise ist die Darstellung auf der Packschablone 16 durch den durchbrochenen Boden 2 hindurch erkennbar und kann genutzt werden, um die Gegenstände entsprechend der Darstellung auf der Packschablone 16 einzulegen oder zu kontrollieren.

Eine noch bessere Betrachtung der Packschablone ist bei der erfindungsgemäßen Ausführungsform möglich, bei der der Boden 2 von den Seitenwänden 3, 4, 5, 6 trennbar ist und entfernt werden kann, wobei dann die Gegenstände über entsprechende Halterungen unmittelbar an den Seitenwänden 3, 4, 5, 6 gelagert werden.

## Patentansprüche

1. Behälter (1) zur Aufnahme von chirurgischen oder medizinischen Gegenständen mit einem Unterteil (9), das einen Boden (2) und Seitenwände (3, 4, 5, 6) aufweist, mit einem auf das Unterteil (9) aufsetzbaren und dieses dadurch verschließende und vom Unterteil (9) wieder abnehmbaren Deckel (10) und mit einer Packschablone (16) zur Anzeige der Gegenstände und ihrer Position im Behälter (1), die an der Innenseite (15) des Deckels (10) angeordnet ist, **dadurch gekennzeichnet, dass** der Boden (2) des Behälters (1) von den Seitenwänden (3, 4, 5, 6) lösbar ist, dass die Gegenstände an Halterungen im Behälter (1) gehalten werden können, die an den Seitenwänden (3, 4, 5, 6) des Unterteils (9) festgelegt sind, und dass das Unterteil (9) nach Abtrennung des Bodens (2) in einer definierten Lage in den Deckel (10) derart einsetzbar ist, dass die Packschablone (16) auf der Innenseite (15) des Deckels (10) unterhalb des Unterteils (9) mit der Packschablone (16) nach oben weisend angeordnet ist.

2. Behälter nach Anspruch 1, **dadurch gekennzeichnet, dass** sich die Packschablone (16) über die gesamte Fläche des Deckels (10) erstreckt.

3. Behälter nach einem der Ansprüche 1 oder 2, **dadurch gekennzeichnet, dass** die Darstellungen der Gegenstände auf der Packschablone (16) unterschiedliche Farben aufweisen.

4. Behälter nach einem der voranstehenden Ansprüche, **dadurch gekennzeichnet, dass** sich das Unterteil (9) beim Einsetzen in den Deckel (10) mit diesem durch eine Rast- oder Klemmverbindung lösbar verbindet.

## Claims

1. Container (1) for receiving surgical or medical articles, having a bottom part (9) that comprises a base (2) and side walls (3, 4, 5, 6), having a lid (10) that is mountable onto and therefore closes the bottom part (9) and is removable from the bottom part (9), and having a packing template (16) disposed on the inside (15) of the lid (10) for indicating the articles and the position thereof in the container (1), **characterized in that** the base (2) of the container (1) is detachable from the side walls (3, 4, 5, 6), that the articles can be held in the container (1) on holders that are fastened to the side walls (3, 4, 5, 6) of the bottom part (9), and that the bottom part (9) after separation of the base (2) can be inserted in a defined position into the lid (10) in such a way that the packing template (16) at the inside (15) of the lid (10) is disposed below the bottom part (9) with the packing template (16) facing upwards.

2. Container according to claim 1, **characterized in that** the packing template (16) extends over the entire surface of the lid (10).

3. Container according to one of claims 1 or 2, **characterized in that** the representations of the articles on the packing template (16) are in different colours.

4. Container according to one of the preceding claims, **characterized in that** the bottom part (9) during insertion into the lid (10) is connected detachably to the lid (10) by means of a detent- or clamping connection.

## Revendications

1. Récipient (1) destiné à recevoir des objets chirurgicaux ou médicaux, comprenant une partie inférieure (9) qui présente un fond (2) et des parois latérales (3, 4, 5, 6), un couvercle (10) qui peut être appliqué sur la partie inférieure (9) en assurant ainsi la fermeture de celle-ci, et peut à nouveau être retiré de la partie inférieure (9), et un gabarit de rangement (16) qui est agencé sur le côté intérieur (15) du couvercle (10), pour visualiser les objets ainsi que leur position dans le récipient (1), **caractérisé en ce que** le fond (2) du récipient (1) peut être détaché des parois latérales (3, 4, 5, 6), **en ce que** les objets peuvent être maintenus sur des supports dans le récipient (1), qui sont fixés aux parois latérales (3, 4, 5, 6) de la partie inférieure (9), et **en ce que** la partie inférieure (9) peut, après détachement du fond (2), être insérée selon une position définie dans le couvercle (10), de manière telle que le gabarit de rangement (16) soit agencé sur le côté intérieur (15) du couvercle (10), sous la partie inférieure (9), avec le gabarit de rangement (16) dirigé vers le haut.

2. Récipient selon la revendication 1, **caractérisé en ce que** le gabarit de rangement (16) s'étend sur la totalité de la surface du couvercle (10).

3. Récipient selon l'une des revendications 1 ou 2, **caractérisé en ce que** les représentations des objets sur le gabarit de rangement (16) présentent des couleurs différentes.

4. Récipient selon l'une des revendications précédentes, **caractérisé en ce que** la partie inférieure (9), lors de son insertion dans le couvercle (10), s'assemble de manière amovible avec celui-ci, par une liaison par encliquetage ou par serrage.
